# EUROPÄISCHE PATENTSCHRIFT

(11) **EP 1 313 735 B1**
(45) Veröffentlichungstag und Bekanntmachung des Hinweises auf die Patenterteilung: **23.04.2008**
(21) Anmeldenummer: 01969490.0
(22) Anmeldetag: 24.07.2001
(51) Int. Cl.: C07D 471/06, C09K 19/34

(54) **FLÜSSIGKRISTALLINE PERYLEN-3,4:9,10-TETRACARBONSÄUREDIIMIDE**
LIQUID CRYSTALLINE 3,4:9,10-PERYLENETETRACARBOXYLIC ACID DIIMIDES
DIIMIDES D'ACIDE PERYLENE-3,4:9,10-TETRACARBOXYLIQUE CRISTALLINS FLUIDES

(30) Priorität: 11.08.2000 DE 10039232
(43) Veröffentlichungstag der Anmeldung: 28.05.2003
(73) Patentinhaber: BASF SE, 67056 Ludwigshafen (DE)
(72) Erfinder: WÜRTHNER, Frank, 89081 Ulm (DE); THALACKER, Christoph, 89077 Ulm (DE)
(86) Internationale Anmeldenummer: PCT/EP2001/008524
(87) Internationale Veröffentlichungsnummer: WO 2002/014318

(56) Entgegenhaltungen:
- DE-A- 3 110 955
- DE-A- 19 620 746
- US-A- 3 294 778
- US-A- 4 378 302
- F. WÜRTHNER ET AL.: "Fluorescent J-type Aggregates and Thermotropic Columnar Mesophases of Perylene Bisimide Dyes" CHEM. EUR. J., Bd. 7, Nr. 10, 10. Mai 2001 (2001-05-10), Seiten 2245-2253, XP002184203
- R. A. CORMIER, B. A. GREGG: "Synthesis and Characterization of Liquid Crystalline Perylene Diimides" CHEM. MATER., Bd. 10, 1998, Seiten 1309-1319, XP002184204

## Beschreibung

Die vorliegende Erfindung betrifft neue Perylen-3,4:9,10-tetracarbonsäurediimide (im folgenden kurz Perylimide genannt) der allgemeinen Formel I 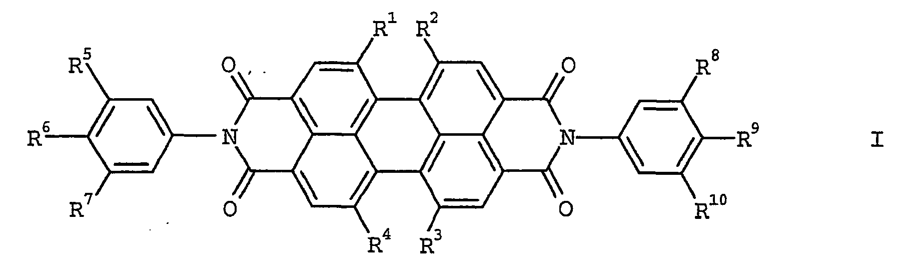 in der die Variablen folgende Bedeutung haben:
- R¹, R², R³ und R⁴: unabhängig voneinander Wasserstoff, Chlor, Brom oder gewünschtenfalls substituiertes Aryloxy, Arylthio, Arylamino, Hetaryloxy oder Hetarylthio;
- R⁵, R⁶, R⁷, R⁸, R⁹ und R¹⁰: unabhängig voneinander Wasserstoff oder C₈-C₂₀ Alkyl, C₈-C₂₀ Alkoxy oder C₈-C₂₀ Alkylthio, dessen Kohlenstoffkette jeweils bis zu vier Doppelbindungen enthalten kann, wobei mindestens vier dieser Reste von Wasserstoff verschieden sind.

Außerdem betrifft die Erfindung die Herstellung dieser Perylimide und ihre Verwendung als flüssigkristalline Materialien für elektronische, optoelektronische und photonische Anwendungen, zum Einfärben von hochmolekularen organischen und von anorganischen Materialien, als Fluoreszenzfarbstoffe und als Laserfarbstoffe sowie als organische Materialien für Solarkollektoren und Elektrolumineszenzanwendungen.

Für eine Vielzahl von technologischen Anwendungen, z.B. für die Anwendung als Ladungstransportmaterial, müssen die verwendeten Materialien nicht nur geeignete molekulare Eigenschaften wie Farbe und Emission sondern auch eine supramolekulare Ordnung, die üblicherweise durch den Kristallverband bestimmt wird, aufweisen.

Besonders interessante Anordnungen von Funktionsmolekülen ergeben sich in flüssigkristallinen Phasen, die gegenüber pigmentären Festkörpern große Vorteile bei der Applikation besitzen. So ermöglicht die in der flüssigkristallinen Phase vorhandene Mobilität der Moleküle die Herstellung von Filmen einheitlicher Schichtdicke und eine durch das Substrat induzierte makroskopische Ordnung. Falls niedrige Viskositäten für den Herstellungsprozeß wünschenswert sind, ist für die Herstellung von Filmen auch eine Temperaturerhöhung über den Schmelzpunkt möglich. Andererseits lassen sich auch über große Temperaturbereiche stabile flüssigkristalline Ordnungen durch Anbringen thermisch oder photochemisch vernetzbarer Gruppen erzielen.

Auf der Basis von Perylimiden sind bislang die folgenden flüssigkristallinen Verbindungen bekannt.

In Chem. Mater. 10, S. 1309 - 1319 (1998) werden Perylimide beschrieben, die an den Imidstickstoffatomen mit Oligoethylenoxysubstituenten als mesogenen Gruppen derivatisiert sind. Diese Gruppen neigen jedoch dazu, Luftfeuchtigkeit aufzunehmen, so daß insbesondere dünne Filme der flüssigkristallinen Phase nicht morphologisch stabil sind.

Angew. Chem. 110, S. 1463 - 1467 (1998) betrifft flüssigkristalline Coronendiimide, die jedoch erst bei Temperaturen über 150°C kolumnare flüssigkristalline Phasen bilden und nur mithilfe von aufwendigen Verfahren ausgehend von Perylenderivaten hergestellt werden können.

Weiterhin werden in der EP-A-422 535 flüssigkristalline Polymere beschrieben, die in den Seitenketten teilweise mit Perylimideinheiten funktionalisiert sind, also keine intrinsisch flüssigkristallinen niedermolekularen Farbstoffe darstellen.

Schließlich sind aus den WO-A-97/22607 und 94/25504 Perylimide bekannt, die im Perylengerüst 1,7-disubstituiert oder 1,6,7,12-tetrasubstituiert sind, sich von den beanspruchten Perylimiden der Formel I jedoch nicht zuletzt durch die Substitution an den Imidstickstoffatomen unterscheiden und nicht flüssigkristallin sind.

Der Erfindung lag die Aufgabe zugrunde, weitere flüssigkristalline Farbstoffe mit vorteilhaften Anwendungseigenschaften bereitzustellen.

Demgemäß wurden die eingangs definierten Perylimide der Formel I gefunden.

Bevorzugte Perylimide der Formel I sind den Unteransprüchen zu entnehmen.

Außerdem wurde ein Verfahren zur Herstellung dieser Perylimide gefunden, welches dadurch gekennzeichnet ist, daß man ein Perylen-3,4:9,10-tetracarbonsäuredianhydrid der allgemeinen Formel II 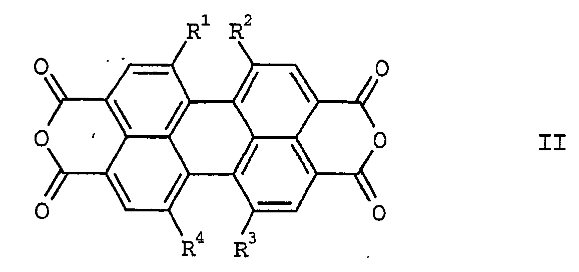 in Gegenwart eines polaren aprotischen Lösungsmittels und eines Imidierungskatalysators mit gleichen oder unterschiedlichen primären Aminen der allgemeinen Formeln III 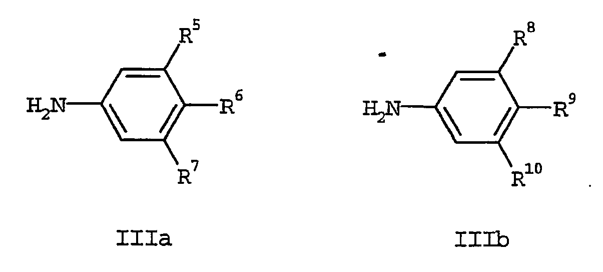 umsetzt.

Weiterhin wurde ein Verfahren zur Herstellung von Perylen-3,4:9,10-tetracarbonsäurediimiden der allgemeinen Formel Ia 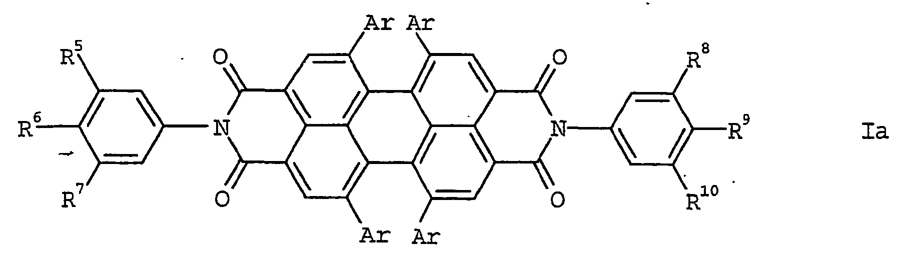 in der Ar gewünschtenfalls substituiertes Aryloxy, Arylthio, Hetaryloxy oder Heterarylthio bedeutet, gefunden, welches dadurch gekennzeichnet ist, daß man 1,6,7,12-Tetrachlorperylen-3,4:9,10-tetracarbonsäuredianhydrid (IIa) in Gegenwart eines polaren aprotischen Lösungsmittels und eines Imidierungskatalysators mit gleichen oder unterschiedlichen primären Aminen der allgemeinen Formeln III 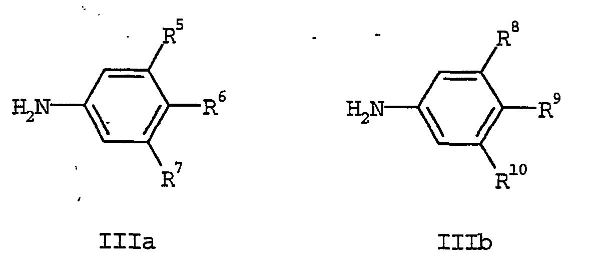 umsetzt und die dabei gebildeten 1,6,7,12-Tetrachlorperylen-3,4:9,10-tetracarbonsäurediimide der allgemeinen Formel I' 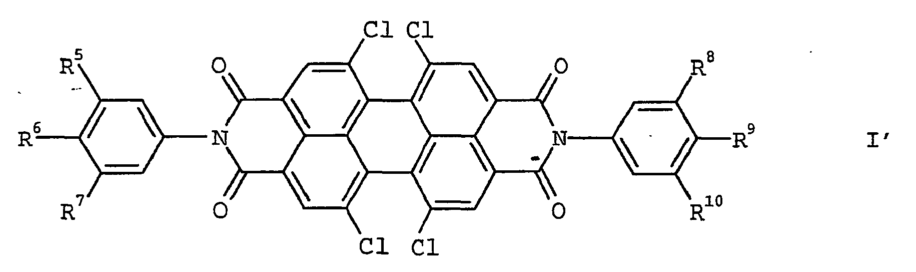 in Gegenwart eines inerten aprotischen Lösungsmittels und einer nicht oder nur schwach nucleophilen Base mit einem aromatischen Alkohol oder Thioalkohol der allgemeinen Formel IV

H-Ar IV

umsetzt.

Schließlich wurde ein Verfahren zur Herstellung von Perylen-3,4:9,10-tetracarbonsäurediimiden der allgemeinen Formel Ib 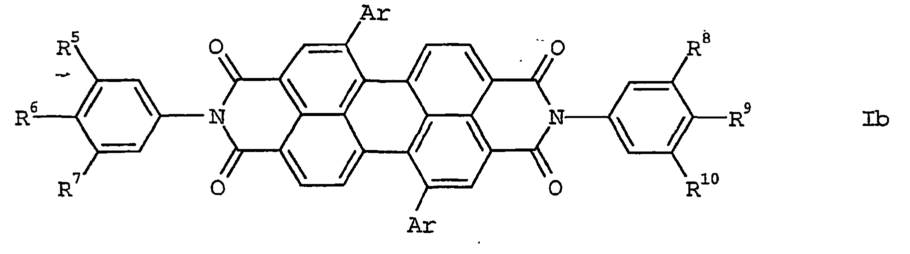 in der Ar gewünschtenfalls substituiertes Aryloxy, Arylthio, Hetaryloxy oder Heterarylthio bedeutet, gefunden, welches dadurch gekennzeichnet ist, daß man 1,7-Dibromperylen-3,4:9,10-tetracarbonsäuredianhydrid (IIb) in Gegenwart eines polaren aprotischen Lösungsmittels und eines Imidierungskatalysators mit gleichen oder unterschiedlichen primären Aminen der allgemeinen Formeln III 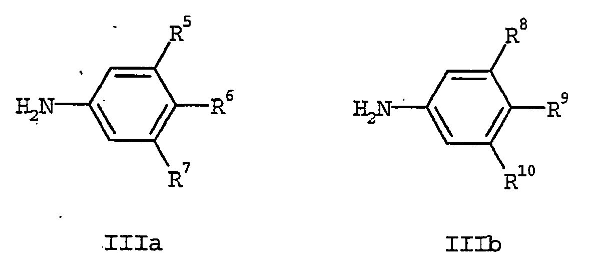 umsetzt und die dabei gebildeten 1,7-Dibromperylen-3,4:9,10-tetracarbonsäurediimide der allgemeinen Formel I" 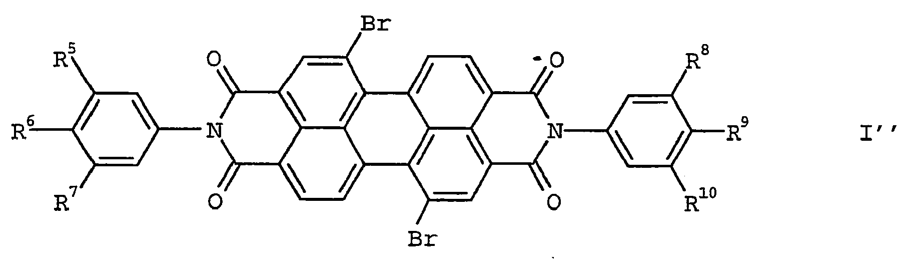 in Gegenwart eines inerten aprotischen Lösungsmittels und einer nicht oder nur schwach nucleophilen Base mit einem aromatischen Alkohol oder Thioalkohol der allgemeinen Formel IV

H-Ar IV

umsetzt.

Nicht zuletzt wurde die Verwendung der Perylimide der Formel I als flüssigkristalline Materialien in elektronischen, optoelektronischen oder photonischen Anwendungen, zum Einfärben von hochmolekularen organischen und von anorganischen Materialien, als Fluoreszenzfarbstoffe und als Laserfarbstoffe sowie als organische Materialien für Solarkollektoren und Elektrolumineszenzanwendungen gefunden.

Im folgenden sollen die Variablen in Formel I näher erläutert werden.

Beispiel für von Wasserstoff verschiedene Reste R¹ bis R⁴ sind Chlor, Brom, Phenoxy, Phenylthio, Phenylamino, 2-Naphthyloxy, 2-Naphthylthio, 2-, 3- und 4-Pyridyloxy, 2-, 3- und 4-Pyridylthio, 2-, 4- und 5-Pyrimidyloxy und 2-, 4- und 5- Pyrimidylthio, wobei Chlor und Brom bevorzugt sind und Phenoxy besonders bevorzugt ist.

Wenn das Perylengerüst substituiert sein soll, dann bedeuten vorzugsweise alle oder nur zwei (insbesondere 1,7-Substitution) der Reste R¹ bis R⁴ gleiche der obengenannten Reste. Die Aryl- und Hetarylreste können dabei jeweils bis zu drei, bevorzugt ein oder zwei, Substituenten tragen.

Beispiele für diese Substituenten sind:
Methyl, Ethyl, Propyl, Isopropyl, Butyl, Isobutyl, tert.-Butyl, Pentyl, Isopentyl, Neopentyl, tert.-Pentyl, Hexyl, 2-Methylpentyl, Heptyl, 1-Ethylpentyl, Octyl, 2-Ethylhexyl, Isooctyl, tert.-Octyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl und Eicosyl (die obigen Bezeichnungen Isooctyl, Isononyl, Isodecyl und Isotridecyl sind Trivialbezeichnungen und stammen von den bei der Oxosynthese erhaltenen Alkoholen), wobei C₁-C₈-Alkylreste und insbesondere tert.-Butyl bevorzugt sind;
2-Methoxyethyl, 2-Ethoxyethyl, 2-Propoxyethyl, 2-Isopropoxyethyl, 2-Butoxyethyl, 2- und 3-Methoxypropyl, 2- und 3-Ethoxypropyl, 2- und 3-Propoxypropyl, 2- und 3- Butoxypropyl, 2- und 4-Methoxybutyl, 2- und 4-Ethoxybutyl, 2- und 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- und 4-Butoxybutyl, 4,8-Dioxadecyl, 3,6,9-Trioxadecyl, 3,6,9-Trioxaundecyl, 3,6,9-Trioxadodecyl, 3,6,9,12-Tetraoxatridecyl und 3,6,9,12-Tetraoxatetradecyl;
2-Methylthioethyl, 2-Ethylthioethyl, 2-Propylthioethyl, 2-Isopropylthioethyl, 2-Butylthioethyl, 2- und 3-Methylthiopropyl, 2- und 3-Ethylthiopropyl, 2- und 3-Propylthiopropyl, 2- und 3- Butylthiopropyl, 2- und 4-Methylthiobutyl, 2- und 4-Ethylthiobutyl, 2- und 4-Propylthiobutyl, 3,6-Dithiaheptyl, 3,6-Dithiaoctyl, 4,8-Dithianonyl, 3,7-Dithiaoctyl, 3,7-Dithianonyl, 4,7-Dithiaoctyl, 4,7-Dithianonyl, 2- und 4-Butylthiobutyl, 4,8-Dithiadecyl, 3,6,9-Trithiadecyl, 3,6,9-Trithiaundecyl, 3,6,9-Trithiadodecyl, 3,6,9,12-Tetrathiatridecyl und 3,6,9,12-Tetrathiatetradecyl;
2-Monomethyl- und 2-Monoethylaminoethyl, 2-Dimethylaminoethyl, 2- und 3-Dimethylaminopropyl, 3-Monoisopropylaminopropyl, 2- und 4-Monopropylaminobütyl, 2- und 4-Dimethylaminobutyl, 6-Methyl-3,6-diazaheptyl, 3,6-Dimethyl-3,6-diazaheptyl, 3,6-Diazaoctyl, 3,6-Dimethyl-3,6-diazaoctyl, 9-Methyl-3,6,9-triazadecyl, 3,6,9-Trimethyl-3,6,9-triazaundecyl, 12-Methyl-3,6,9,12-tetraazatridecyl und 3,6,9,12-Tetramethyl-3,6,9,12-tetraazatridecyl;
Propan-2-on-1-yl, Butan-3-on-1-yl, Butan-3-on-2-yl und 2-Ethylpentan-3-on-1-yl;
2-Methylsulfonylethyl, 2-Ethylsulfonylethyl, 2-Propylsulfonylethyl, 2-Isopropylsulfonylethyl, 2-Butylsulfonylethyl, 2- und 3-Methylsulfonylpropyl, 2- und 3-Ethylsulfonylpropyl, 2- und 3-Propylsulfonylpropyl, 2- und 3-Butylsulfonylpropyl, 2- und 4-Methylsulfonylbutyl, 2- und 4-Ethylsulfonylbutyl, 2- und 4-Propylsulfonylbutyl und 4-Butylsulfonylbutyl;
Carboxymethyl, 2-Carboxyethyl, 3-Carboxypropyl, 4-Carboxybutyl, 5-Carboxypentyl, 6-Carboxyhexyl, 8-Carboxyoctyl, 10-Carboxydecyl, 12-Carboxydodecyl und 14-Carboxytetradecyl;
Methylcarboxymethyl, Ethylcarboxymethyl, Propylcarboxymethyl, Butylcarboxymethyl, Pentylcarboxymethyl, Hexylcarboxymethyl, Methyl-2-carboxyethyl, Ethyl-2-carboxyethyl, Propyl-2-carboxyethyl, Butyl-2-carboxyethyl, Pentyl-2-carboxyethyl, Hexyl-2-carboxyethyl, Methyl-3-carboxypropyl, Ethyl-3-carboxypropyl, Propyl-3-carboxypropyl, Butyl-3-carboxypropyl, Pentyl-3-carboxy-propyl, Hexyl-3-carboxypropyl, Methyl-4-carboxybutyl, Methyl-5-carboxypentyl, Methyl-6-carboxyhexyl, Methyl-8-carboxyroctyl, Methyl-10-carboxydecyl, Methyl-12-carboxydedecyl und Methyl-14-carboxytetradecyl;
Sulfomethyl, 2-Sulfoethyl, 3-Sulfopropyl, 4-Sulfobutyl, 5-Sulfopentyl, 6-Sulfohexyl, 8-Sulfooctyl, 10-Sulfodecyl, 12-Sulfododecyl und 14-Sulfotetradecyl;
Methylsulfomethyl, Ethylsulfomethyl, Propylsulfomethyl, Butylsulfomethyl, Pentylsulfomethyl, Hexylsulfomethyl, Methyl-2-sulfoethyl, Ethyl-2-sulfoethyl, Propyl-2-sulfoethyl, Butyl-2-sulfoethyl, Pentyl-2-sulfoethyl, Hexyl-2-sulfoethyl, Methyl-3-sulfopropyl, Ethyl-3-sulfopropyl, Propyl-3-sulfopropyl, Butyl-3-sulfopropyl, Pentyl-3-sulfopropyl, Hexyl-3-sulfopropyl, Methyl-4-sulfobutyl, Methyl-5-sulfopentyl, Methyl-6-sulfohexyl, Methyl-8-sulfooctyl, Methyl-10-sulfodecyl, Methyl-12-sulfododecyl und Methyl-14-sulfotetradecyl;
2-Hydroxyethyl, 2- und 3-Hydroxypropyl, 1-Hydroxyprop-2-yl, 2- und 4-Hydroxybutyl, 1-Hydroxybut-2-yl und 8-Hydroxy-4-oxaoctyl;
2-Cyanoethyl, 3-Cyanopropyl, 2-Methyl-3-ethyl-3-cyanopropyl, 7-Cyano-7-ethylheptyl und 4-Methyl-7-methyl-7-cyanoheptyl;
Methoxy, Ethoxy, Propoxy, Isopropoxy, Butoxy, Isobutoxy, sec.-Butoxy, tert-Butoxy, Pentyloxy, Isopentyloxy, Neopentyloxy, tert.-Pentyloxy, Hexyloxy und 2-Methylpentyloxy;
Cyclopentyl, Cyclohexyl, Cycloheptyl, Cyclooctyl, Morpholinyl, Pyrrolidinyl und Piperidyl.

Beispiele für besonders bevorzugte substituierte aromatische Reste R¹ bis R⁴ sind 4- und 3-tert.-Butylphenoxy, 4-Cyclohexylphenoxy, 4-Propoxyphenoxy, 4-Butoxyphenoxy, 4-Hexyloxyphenoxy und 1,1,3,3-Tetramethylbutylphenoxy.

Mindestens vier der die Phenylreste an den Imidstickstoffatomen substituierenden Reste R⁵ bis R¹⁰ sind von Wasserstoff verschieden, wobei vorzugsweise beide Phenylreste jeweils zwei der von Wasserstoff verschiedenen Substituenten tragen. Dabei ist sowohl 3,5- als auch 3,4-Disubstitution möglich. Bevorzugt sind jedoch alle Reste R⁵ bis R¹⁰ von Wasserstoff verschieden. Sie können dabei gleich oder verschieden sein. Vorzugsweise sind jedoch mindestens die an einem Phenylrest befindlichen Reste gleich. Besonders bevorzugt sind Perylimide der Formel I, in der beide Phenylreste dieselben Substituenten tragen.

Die Alkylketten der Reste R⁵ bis R¹⁰ können linear oder verzweigt sein und weisen in der Regel 8 bis 20, bevorzugt 10 bis 14, Kohlenstoffatome auf.

Neben den bereits oben genannten Alkylresten seien für die Reste R⁵ bis R¹⁰ beispielhaft folgende geeignete Alkoxy- und Alkylthioreste genannt:
Octyloxy, 2-Ethylhexyloxy, Isooctyloxy, Nonyloxy, Isononyloxy, Decyloxy, Isodecyloxy, Undecyloxy, Dodecyloxy, Tridecyloxy, Tetradecyloxy, Pentadecyloxy, Hexadecyloxy, Heptadecyloxy, Octadecyloxy, Nonadecyloxy und Eicosyloxy;
Octylthio, 2-Ethylhexylthio, Isooctylthio, Nonylthio, Isononylthio, Decylthio, Isodecylthio, Undecylthio, Dodecylthio, Tridecylthio, Tetradecylthio, Pentadecylthio, Hexadecylthio, Heptadecylthio, Octadecylthio,Nonadecylthio und Eicosylthio.

Außerdem können die Alkylketten der Reste R⁵ bis R¹⁰ ein bis vier, insbesondere drei, Doppelbindungen enthalten. Bei diesen ungesättigten Resten sind vor allem solche Reste geeignet, die sich von natürlich vorkommenden Terpenkohlenwasserstoffen und Terpenalkoholen und von der Alkoholen ungesättigter Fettsäuren ableiten. Besonders geeignete Alkenyloxyreste werden z.B. von Geraniol, Nerol, Linalool und Citronellol sowie den Alkoholen von Ölsäure, Linolsäure und Linolensäure gebildet.

Beispiele für besonders bevorzugte die Imidstickstoffatome substituierende Phenylreste sind 3,4,5-Tridodecyloxyphenyl, 3,4,5-Tridecyloxyphenyl, 3,5- und 3,4-Didodecyloxyphenyl.

Die erfindungsgemäßen Perylimide der Formel I können vorteilhaft nach dem ebenfalls erfindungsgemäßen Verfahren hergestellt werden, bei dem die wie gewünscht substituierten Perylen-3,4:9,10-tetracarbonsäuredianhydride der Formel II in Gegenwart eines polaren aprotischen Lösungsmittels und eines Imidierungskatalysators mit gleichen oder unterschiedlichen primären Aminen der Formeln III umgesetzt werden.

Als polares aprotisches Lösungsmittel eignen sich dabei vor allem aprotische Stickstoffheterocyclen, wie Pyridin, Pyrimidin, Imidazol, Chinolin, Isochinolin, Chinaldin, N-Methylpiperidin, N-Methylpiperidon und N-Methylpyrrolidon, Carbonsäureamide, wie Dimethylformamid und Dimethylacetamid, und Tetraalkylharnstoffe, wie Tetramethylharnstoff, wobei Chinolin besonders bevorzugt ist.

Die Menge an Lösungsmittel ist an sich nicht kritisch. Es können 5 bis 120 g Lösungsmittel je g Perylen-3,4:9,10-tetracarbonsäuredianhydrid (II) zum Einsatz kommen.

Als Imidierungskatalysatoren eignen sich insbesondere Lewis-saure Salze organischer und anorganischer Säuren mit Metallen wie Zink, Eisen, Kupfer und Magnesium sowie die Oxide dieser Metalle, z.B. Zinkacetat, Zinkpropionat, Zinkoxid, Eisen (II) acetat, Eisen(III)-chlorid, Eisen (II) sulfat, Kupfer (II)acetat, Kupfer (II)oxid und Magnesiumacetat, wobei Zinkacetat besonders bevorzugt ist. Selbstverständlich kann man auch Mischungen der genannten Katalysatoren verwenden. Bevorzugte Mengen an den Metallkatalysatoren betragen etwa 20 bis 100 mol-%, bezogen auf (II).

Man kann auch die Säuren selbst, also z.B. organische Säuren, insbesondere C₁-C₃-Carbonsäuren wie Ameisensäure, Essigsäure und Propionsäure, und anorganische Säuren wie Phosphorsäure, jeweils bevorzugt in möglichst konzentrierter Form, als Imidierungskatalysatoren einsetzen. Die Säuren dienen dabei gleichzeitig als Lösungsmittel oder als Cosolvens und kommen daher üblicherweise im Überschuß zum Einsatz.

Üblicherweise liegt das Molverhältnis von primärem Amin (III) zum Perylen-3,4:9,10-tetracarbonsäuredianhydrid (II) bei etwa 2:1 bis 4:1, vorzugsweise bei etwa 2,2:1 bis 3:1.

Die Reaktionstemperatur beträgt im allgemeinen 60 bis 250°C, bevorzugt 100 bis 230°C, besonders bevorzugt 160 bis 200°C.

Es empfiehlt sich, die Umsetzung unter Schutzgasatmosphäre (vorzugsweise Argon oder auch Stickstoff) durchzuführen.

In der Regel ist es nicht erforderlich, bei diesem erfindungsgemäßen Verfahren unter Druck zu arbeiten.

Üblicherweise ist die Umsetzung in 1 bis 4 h beendet.

Verfahrenstechnisch geht man zweckmäßigerweise wie folgt vor:

Man legt Perylen-3,4:9,10-tetracarbonsäuredianhydrid (II), Lösungsmittel und Katalysator vor, fügt das Amin (III) unter Rühren bei Raumtemperatur zu, spült die Apparatur etwa 10 min mit Argon, erhitzt das Gemisch unter Rühren auf die Reaktionstemperatur und hält es etwa 2 bis 3 h bei dieser Temperatur. Nach dem Abkühlen auf Raumtemperatur filtriert man das Reaktionsprodukt ab, wäscht zunächst mit Wasser und dann mit einem aliphatischen Alkohol wie Methanol.

Zur nachfolgenden Reinigung kann das Produkt umkristallisiert werden (z.B. Lösen in einem organischen Lösungsmittel wie Dichlormethan und Wiederausfällen mit einem aliphatischen Alkohol wie Methanol) oder einer Säulenchromatographie (z.B. Kieselgel/Dichlormethan) unterzogen werden.

Soll das Perylen-3,4:9,10-tetracarbonsäuredianhydrid (II) mit zwei verschiedenen Aminen (IIIa) und (IIIb) umgesetzt werden, so wird die Umsetzung zweckmäßigerweise stufenweise vorgenommen, indem zunächst das Amin (IIIa) in einer Menge von etwa 0,8 bis 1 mol pro mol (II) und nach beendeter Bildung des Monoimids das Amin (IIIb) in einer Menge von etwa 1 bis 2 mol pro mol (II) zur Bildung des Diimids eingesetzt wird (vgl. Adv. Mater. 11, S. 754 - 758 (1999)).

Mit Hilfe dieses erfindungsgemäßen Verfahrens können die Perylimide der Formel I vorteilhaft in hoher Reinheit (Wertgehalt in der Regel ≥ 95%) und guter Ausbeute (im allgemeinen von 60 bis 95%) erhalten werden.

Die als Ausgangsstoffe für dieses erfindungsgemäße Herstellungsverfahren dienenden Perylen-3,4:9,10-tetracarbonsäuredianhydride (II) sind an sich bekannt oder können nach bekannten Methoden ausgehend von 1,6,7,12-Tetrachlorperylen-3,4:9,10-tetracarbonsäuredianhydrid (IIa) bzw. 1,7-Dibromperylen-3,4:9,10-tetracarbonsäuredianhydrid (IIb) hergestellt werden (vgl. WO-A-97/22607 und EP-A-227 980).

Eine weitere Möglichkeit zur Herstellung der (het)aryloxy- und (het)arylthiosubstituierten Perylimide der Formeln Ia und Ib besteht gemäß den weiteren erfindungsgemäßen Verfahren darin, in einem ersten Schritt 1,6,7,12-Tetrachlorperylen-3,4:9,10-tetracärbonsäuredianhydrid (IIa) bzw. 1,7-Dibromperylen-3,4:9,10-tetracarbonsäuredianhydrid (IIb) in Gegenwart eines polaren aprotischen Lösungsmittels und eines Imidierungskatalysators mit gleichen oder unterschiedlichen primären Aminen der Formeln III umzusetzen und die dabei gebildeten 1,6,7,12-Tetrachlorperylen-3,4:9,10-tetracarbonsäurediimide der Formel I' bzw. 1,7-Dibromperylen-3,4:9,10-tetracarbonsäurediimide der Formel I" in einem zweiten Schritt in Gegenwart eines inerten aprotischen Lösungsmittels und einer nicht oder nur schwach nucleophilen Base mit einem aromatischen (Thio)Alkohol der Formel IV weiterumzusetzen.

Der erste Schritt dieser erfindungsgemäßen Herstellungsverfahren kann analog zu dem oben beschriebenen Verfahren vorgenommen werden.

Für den zweiten Schritt dieser Herstellungsverfahren, bei dem wie in der WO-A-97/22607 beschrieben vorgegangen werden kann, eignen sich als inertes aprotisches Lösungsmittel vor allem Stickstoffheterocyclen wie Pyridin, Pyrimidin, Chinolin, Isochinolin, Chinaldin und insbesondere N-Methylpyrrolidon als inertes aprotisches Reaktionsmedium.

Die Menge an Lösungsmittel ist an sich nicht kritisch, üblicherweise kommen 10 bis 50 g, vorzugsweise 25 bis 35 g, Lösungsmittel je g Perylimid der Formel I' bzw. I" zum Einsatz.

Als Basen sind Alkalimetallhydroxide, z.B. Natriumhydroxid und Kaliumhydroxid, und vor allem Alkalimetallcarbonate, z.B. Natriumcarbonat und Kaliumcarbonat, bevorzugt.

In der Regel werden 2 bis 3, vorzugsweise 2,2 bis 2,5, Moläquivalente Base je mol Perylimid der Formel I' bzw. I" eingesetzt.

Das Molverhältnis von aromatischem (Thio)Alkohol (IV) zum Perylimid der Formel I' bzw. I" liegt in der Regel bei 2:1 bis 3:1, vorzugsweise bei 2,0:1 bis 2,2:1.

Die Reaktionstemperatur beträgt üblicherweise 60 bis 180°C, vor allem 80 bis 140°C.

Es empfiehlt sich ebenfalls, unter einer Schutzgasatmosphäre zu arbeiten.

Üblicherweise ist die Umsetzung in 1 bis 5 h, vor allem in 1 bis 2 h, beendet.

Verfahrenstechnisch geht man in diesem zweiten Schritt zweckmäßigerweise wie folgt vor:

Man legt eine gerührte Suspension von Perylimid (I') bzw. (I''), (Thio)Alkohol (IV) und Base im Lösungsmittel vor und erhitzt unter Schutzgas 1 bis 2 h auf die Reaktionstemperatur. Nach dem Abkühlen auf Raumtemperatur trägt man das Reaktionsgemisch in das etwa 3fache Volumen an einer verdünnten anorganischen Säure, z.B. 5 bis 10 gew.-%iger Salzsäure, ein, filtriert das ausgefallene Reaktionsprodukt ab, wäscht mit Wasser bis zum neutralen Ablauf und trocknet im Vakuum.

In der Regel haben die so erhaltenen Perylimide der Formel Ia bzw. Ib bereits einen Wertgehalt ≥ 95%, so daß auf eine weitere Reinigung verzichtet werden kann. Ist eine zusätzliche Reinigung jedoch erwünscht, so kann diese wie bei dem bereits beschriebenen weiteren Herstellungsverfahren vorgenommen werden.

Die erfindungsgemäßen Perylimide der Formel I eignen sich aufgrund der Ausbildung stabiler flüssigkristalliner Phasen hervorragend für eine Vielzahl von Anwendungen, insbesondere für elektronische, optoelektronische und photonische Anwendungen, z.B. als Ladungstransportmaterialien in Leuchtdioden und photovoltaischen Dioden, Photoleitern und Transistoren. Außerdem sind sie als Fluoreszenzfarbmittel zum Einfärben von hochmolekularen organischen Materialien (z.B. Polyolefinen) und anorganischen Materialien geeignet und können als Laserfarbstoffe Anwendung finden.

Nicht zuletzt können sie als organische Materialien für Solarkollektoren und für Elektrolumineszenzanwendungen, z.B. in Displays, eingesetzt werden.

### Beispiele

### A) Herstellung von erfindungsgemäßen Perylimiden

### Beispiel 1

### N,N'-Di(3,4,5-tridodecyloxyphenyl)perylen-3,4:9,10-tetracarbonsäurediimid:

Ein Gemisch von 0,12 g (0,3 mmol) Perylen-3,4:9,10-tetracarbonsäuredianhydrid, 0,58 g (0,9 mmol) 3,4,5-Tridodecyloxyanilin, 0,04 g (0,2 mmol) Zinkacetat und 12 ml Chinolin wurde 3 h unter Argon auf 180°C erhitzt.

Nach Abkühlen auf Raumtemperatur wurde die Reaktionsmischung unter Rühren in 100 ml In Salzsäure eingegossen. Der erhaltene rote Niederschlag wurde abgesaugt und zunächst mit Wasser und dann mit Methanol gewaschen. Anschließend wurde das Produkt durch Lösen in Dichlormethan und Ausfällen durch Zugabe von Methanol gereinigt und bei 70°C im Feinvakuum getrocknet.

Es wurden 0,44 g Produkt mit einem Wertgehalt von 99% erhalten, was einer Ausbeute von 89% entspricht.

### Analytische Daten:

Elementaranalyse für C₁₀₈H₁₆₂N₂O₁₀ (1648,5) (Gew.-% ber./gef.): C: 78,69/78,50; H: 9,91/9,81; N: 1,70/1,62;
1H-NMR (200 MHz, CDCl₃, 25°C, TMS) : δ = 8,54 (d, 3J(H,H)=7,8 Hz, 4H; H2,5,8,11), 8,21 (d, 3J(H,H)=7,8 Hz, 4H; H1,6,7,12), 6,63 (s, 4H; ArH), 4,03 (t, 4H; OCH₂), 3,86 (t, 8H; OCH₂), 1,76 (m, 12H), 1,6-1,2 (m, 108H), 0,85 (m, 18H) ppm;
UV/Vis (CH₂Cl₂) : λₘₐₓ (ε) = 527 (89 600), 490 (59 500), 459 nm (22 300 mol⁻¹ dm³ cm⁻¹).

### Beispiel 2

### N,N'-Di(3,4,5-tridodecyloxyphenyl)-1,7-di(4-tert.-butylphenoxy)perylen-3,4:9,10-tetracarbonsäurediimid:

Es wurde analog Beispiel 1 vorgegangen, jedoch wurden 0,34 g (0,5 mmol) 1,7-Di(4-tert.-butylphenoxy)perylen-3,4:9,10-tetracarbonsäuredianhydrid und 0,97 g (1,5 mmol) 3,4,5-Tridodecyloxyanilin und 0,05 g (0,3 mmol) Zinkacetat eingesetzt.

Die.Reinigung des dunkelroten Produkts erfolgte durch Säulenchromatographie mit Dichlormethan an Kieselgel.

Es wurden 0,58 g Produkt mit einem Wertgehalt von 99% erhalten, was einer Ausbeute von 60% entspricht.

### Analytische Daten:

Elementaranalyse für C₁₂₈H₁₈₆N₂O₁₂ (1944,6) (Gew.-% ber./gef.): C: 79,05/79,00; H: 9,64/9,77; N: 1,44/1,49;
1H-NMR (200 MHz, CDCl₃, 25°C, TMS): δ = 9,52 (d, 3J(H,H)=8,4 Hz, 2H; H6,12), 8,53 (d, 3J(H,H)=8,4 Hz, 2H; H5,11), 8,27 (s, 2H; H2,8), 7,47 (d, 3J(H,H)=8,7 Hz, 4H; H3'), 7,10 (d, 3J(H,H)=8,7 Hz, 4H; H2'), 6,52 (s, 4H; ArH), 4,00 (t, 4H; OCH₂), 3,83 (t, 8H; OCH₂), 1,74 (m, 12H), 1,6-1,2 (m, 108H), 1,36 (s, 18H; tert.Bu), 0,87 (m, 18H) ppm;
DV/Vis (CH₂Cl₂): λₘₐₓ (ε) = 546 (57 500), 511 (39 600), 402 nm (11 600 mol⁻¹ dm³ cm⁻¹) .

### Beispiel 3

### N,N'-Di(3,4,5-tridodecyloxyphenyl)-1,6,7,12-tetraphenoxyperylen-3,4:9,10-tetracarbonsäurediimid:

Es wurde analog Beispiel 1 vorgegangen, jedoch wurden 0,19 g (0,25 mmol) 1,6,7,12-Tetraphenoxyperylen-3,4:9,10-tetracarbonsäuredianhydrid, 0,48 g (0,75 mmol) 3,4,5-Tridodecyloxyanilin, 0,04 g (0,2 mmol) Zinkacetat und 10 ml Chinolin eingesetzt.

Die Reinigung des violetten Niederschlags erfolgte analog Beispiel 2.

Es wurden 0,35 g Produkt mit einem Wertgehalt von 99% erhalten, was einer Ausbeute von 69% entspricht.

### Analytische Daten:

Elementaranalyse für C₁₃₂H₁₇₈N₂O₁₄ (2016,9) (Gew.-% ber./gef.): C: 78,61/78,40; H: 8,90/9,02; N: 1,39/1,40;
1H-NMR (400 MHz, CDCl₃, 25°C, TMS): δ = 8,22 (s, 4H; H2,5,8,11), 7,25 (t, 3J(H,H)=8,0 Hz, 8H; H3'), 7,10 (t, 3J(H,H)=7,4 Hz, 4H; H4'), 6,95 (d, 3J(H,H)=8,1 Hz, 8H; H2'), 6,41 (s, 4H; ArH), 3,99 (t, 4H; OCH₂), 3,89 (t, 8H; OCH₂), 1,76 (m, 12H), 1,5-1,2 (m, 108H), 0,87 (m, 18H) ppm;
UV/Vis (CH₂Cl₂): λₘₐₓ(ε) = 574 (51 500), 535 (32 500), 445 (16 400) nm (mol⁻¹ dm³ cm⁻¹).

### Beispiel 4

### N,N'-Di(3,4,5-tridodecyloxyphenyl)-1,6,7,12-tetra(4-tert.-butylphenoxy)perylen-3,4:9,10-tetracarbänsäurediimid:

Es wurde analog Beispiel 1 vorgegangen, jedoch wurden 0,25 g (0,25 mmol) 1,6,7,12-Tetra(4-tert.-butylphenoxy)perylen-3,4:9,10-tetracarbonsäuredianhydrid, 0,48 g (0,75 mmol) 3,4,5-Tridodecyloxyanilin, 0,04 g (0,2 mmol) Zinkacetat und 10 ml Chinolin eingesetzt.

Es wurden 0,52 g Produkt mit einem Wertgehalt von 98% erhalten, was einer Ausbeute von 92% entspricht.

### Analytische Daten:

Elementaranalyse für C₁₄₈H₂₁₀N₂O₁₄ (2241,3) (Gew.-% ber./gef.): C: 79,31/78,91; H: 9,44/9,27; N: 1,25/1,27;
1H-NMR (200 MHz, CDCl₃, 25°C, TMS): δ = 8,23 (s, 4H; H2,5,8,11), 7,24 (d, 3J (H,H)=8, 7 Hz, 8H; H3'), 6,86 (d, 3J(H,H) =8, 7 Hz, 8H; H2'), 6,41 (s, 4H; ArH), 3,98 (t, 4H; OCH₂), 3,89 (t, 8H; OCH₂), 1,75 (m, 12H), 1,6-1,2 (m, 108H), 1,26 (s, 36H; tert.Bu), 0,87 (m, 18H) ppm;
UV/Vis (CH₂Cl₂): λₘₐₓ (ε) = 580 (42 500), 542 (28 000), 452 nm (15 800 mol⁻¹ dm³ cm⁻¹).

### B) Untersuchung der Eigenschaften der hergestellten Perylimide

### Beispiel 5

### Charakterisierung der flüssigkristallinen Eigenschaften des Perylimids aus Beispiel 1:

Bei Erhitzen einer Probe der Verbindung über die Klärtemperatur von 376°C und anschließendem langsamem Abkühlen wurde im Polarisationsmikroskop bei gekreuzten Polarisatoren die Ausbildung einer für columnare Mesophasen typischen sphärulitischen Textur mit großen pseudoisotropen Bereichen beobachtet. Obwohl die Probe bei tieferen Temperaturen glasartig erstarrte, war im Polarisationsmikroskop und auch durch DSC (Heiz/Kühlrate: 10°C/min) kein weiterer Phasenübergang festzustellen. Beim erneuten Aufheizen blieb die Textur unabhängig von der Heizrate bis zum Klärpunkt erhalten.

Das Weitwinkel-Röntgenbeugungs-Diffraktogramm (WAXS; Cu-Ka, Nigefiltert) einer auf Raumtemperatur abgekühlten Probe zeigte einen scharfen Reflex bei 2ϑ = 3,14° sowie eine diffuse Halo bei 2ϑ = 20°. Ein zusätzliches Beugungsexperiment an einer orientierten Probe zeigte eine hexagonale Anordnung der Reflexe 1. Ordnung und ließ somit auf eine ungeordnete hexagonale diskotische Mesophase D_{hd} mit einer Gitterkonstanten von 32,5 Å und einem Molekül pro Columnensegment schließen.

### Beispiel 6

### Charakterisierung der flüssigkristallinen Eigenschaften des Perylimids aus Beispiel 4:

Bei Erhitzen einer Probe der Verbindung über die Klärtemperatur von 346°C und anschließendem langsamem Abkühlen wurde im Polarisationsmikroskop bei gekreuzten Polarisatoren die Ausbildung einer für columnare Mesophasen typischen sphärulitischen Textur mit großen pseudoisotropen Bereichen beobachtet. Obwohl die Probe bei tieferen Temperaturen glasartig erstarrte, war im Polarisationsmikroskop und auch durch DSC (Heiz/Kühlrate: 10°C/min) kein weiterer Phasenübergang festzustellen. Beim erneuten Aufheizen blieb die Textur unabhängig von der Heizrate bis zum Klärpunkt erhalten.

Das Weitwinkel-Röntgenbeugungs-Diffraktogramm (WAXS; Cu-Ka, Nigefiltert) einer auf Raumtemperatur abgekühlten Probe zeigte zwei scharfe Reflexe bei 2ϑ = 3,57° und 2ϑ = 6,12° sowie eine diffuse Halo bei 2ϑ = 20°, die auf eine ungeordnete hexagonale diskotische Mesophase D_{hd} mit einer Gitterkonstanten von 28,7 Å und einem Molekül pro Columnensegment schließen ließen.

### Beispiel 7

### Charakterisierung der Fluoreszenzeigenschaften des Perylimids aus Beispiel 4:

In Dichlormethan wurde eine intensive Fluoreszenz mit einem Emissionsmaximum bei 616 nm beobachtet. Die für eine 8.10⁻⁷ molare Lösung bestimmte Fluoreszenzquantenausbeute betrug 21%.

In aliphatischen Lösungsmitteln wie Methylcyclohexan war bei höheren Konzentrationen sowohl in den Absorptions- als auch in den Emissionsspektren eine starke Aggregation zu beobachten. Dabei verschob sich das Absorptionsmaximum um 19 nm und das Emissionsmaximum um 40 nm zu längeren Wellenlängen, was sich durch excitonische Wechselwirkungen der aggregierten Chromophore (J-Aggregat) erklären läßt. Dennoch wurde für die Fluoreszenzintensität eine nahezu lineare Abhängigkeit von der Konzentration beobachtet.

Das Fluoreszenzspektrum eines auf Quarzglas abgeschiedenen Films der Verbindung war mit den für konzentrierte Lösungen erhaltenen nahezu identisch und wies ebenso eine rote Farbe mit intensiver Fluoreszenz auf.

## Patentansprüche

1. Perylen-3,4:9,10-teracarbonsäurediimide der allgemeinen Formel 1 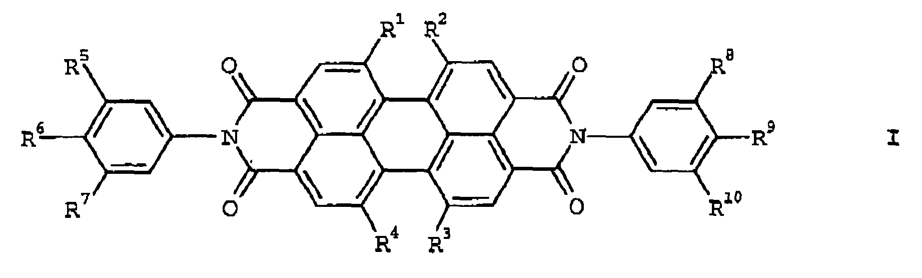 in der die Variablen folgende Bedeutung haben:
R¹, R², R³ und R⁴ unabhängig voneinander Wasserstoff, Chlor, Brom oder gewünschtenfalls substituiertes Aryloxy, Arylthio, Arylamino, Hetaryloxy oder Hetarylthio;
R⁵, R⁶, R⁷, R⁸, R⁹ und R¹⁰ unabhängig voneinander Wasserstoff oder C₈-C₂₀ Alkyl, C₈-C₂₀ Alkoxy oder C₈-C₂₀ Alkylthio, dessen Kohlenstoffkette jeweils bis zu vier Doppelbindungen enthalten kann, wobei mindestens vier dieser Reste von Wasserstoff verschieden sind.

2. Perylen-3,4:9,10-tetracarbibsayreduunude der Formel I nach Anspruch 1, in der die Variablen folgende Bedeutung haben:
R¹, R², R³ und R⁴ unabhängig voneinander Wasserstoff, Chlor, Brom oder Phenoxy, das bis zu drei der folgenden Substituenten tragen kann: C₁-C₂₀-Alkyl, dessen Kohlenstoff kette durch eine oder mehrere Gruppierungen -O-, -S-, -NR¹¹-, -CO- und/oder -SO₂- unterbrochen sein kann und/oder das durch -COOR¹¹, -SO₃R¹¹, Hydroxy, Cyano, C₁-C₆-Alkoxy, C₅-C₈-Cycloalkyl oder einen über ein Stickstoffatom gebundenen, 5- bis 7-gliedrigen heterocyclischen Rest, der weitere Heteroatome enthalten kann, ein- oder mehrfach substituiert sein kann; C₁-C₆-Alkoxy; Cyano; Hydroxy; Halogen; Nitro, -COOR¹¹ oder -SO₃R¹¹;
R⁵, R⁶, R⁷, R⁸, R⁹ und R¹⁰ unabhängig voneinander C₈-C₂₀-Alkyl, C₈-C₂₀-Alkoxy oder C₉-C₂₀-Alkylthio, dessen Kohlenstoffkette jeweils bis zu vier Doppelbindungen enthalten kann;
R¹¹ Wasserstoff oder C₁-C₆-Alkyl.

3. Perylen-3,4,9,10-tetracarbonsäurediimide der Formel I nach Anspruch 1, in der,die Variablen folgende Bedeutung haben:
R¹, R², R³ und R⁴ unabhängig voneinander Wasserstoff oder Phenoxy, das einfach durch C₁-C₈-Alkyl substituiert sein kann;
R⁵, R⁶, R⁷, R⁸, R⁹ und R¹⁰ C₁₀-C₁₄-Alkyl.

4. Verfahren zur Herstellung von Perylen-3,4:9,10-tetracarbonsäurediimiden der Formel I gemäß den Ansprüchen 1 bis 3, **dadurch gekennzeichnet, daß** man ein Perylen-3,4:9,10-tetracarbonsäuredianhydrid der allgemeinen Formel II 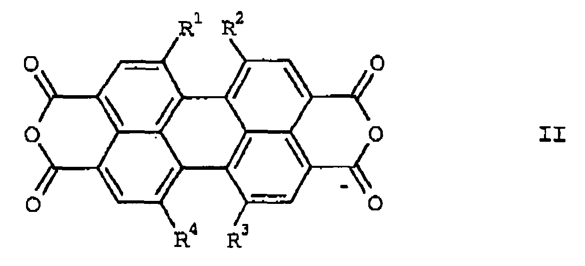 in Gegenwart eines polaren aprotischen Lösungsmittels und eines Imidierungskatalysators mit gleichen oder unterschiedlichen primären Aminen der allgemeinen Formeln III 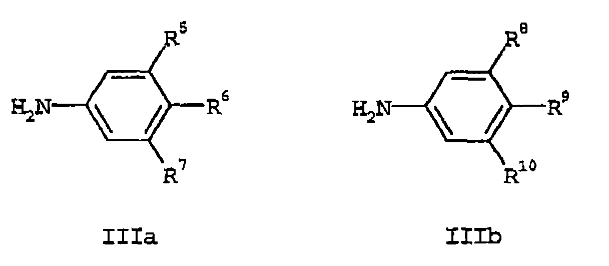 umsetzt.

5. Verfahren zur Herstellung von Perylen-3,4:9,10-tetracarbonsäurediimiden der allgemeinen Formel Ia 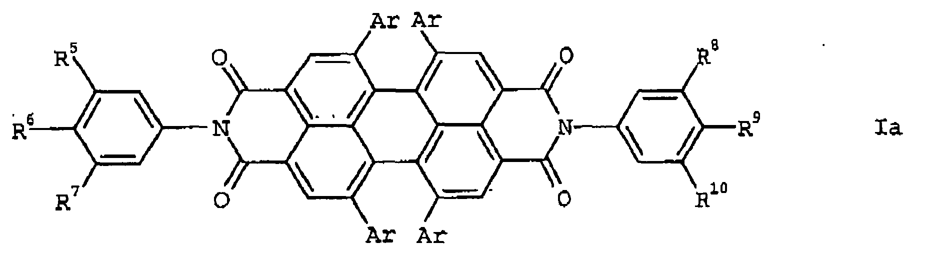 in der Ar gewünschtenfalls substituiertes Aryloxy, Arylthio, Hetaryloxy oder Heterarylthio bedeutet, **dadurch gekennzeichnet, daß** man 1,6,7,12-Tetrachorperylen-3,4:9,10-tetracarbonsäuredianhydrid (IIa) in Gegenwart eines polaren aprotischen Lösungsmittels und eines Imidierungskatalysators mit gleichen oder unterschiedlichen primären Aminen der allgemeinen Formeln III 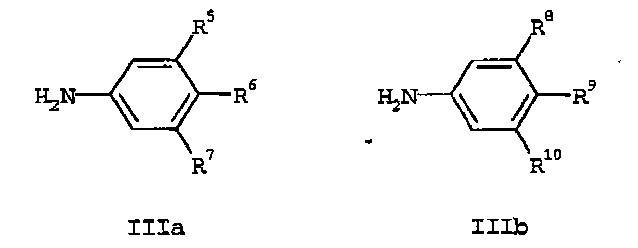 umsetzt und die dabei gebildeten 1,6,7,12-Tetrachlorperylen-3,4:9,10-tetracarbonsäurediimide der allgemeinen Formel I' 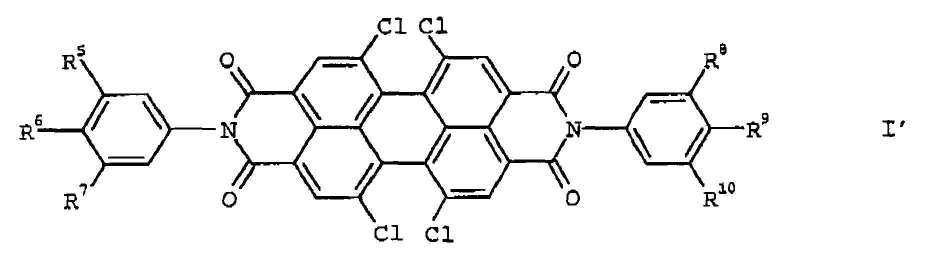 in Gegenwart eines inerten aprotischen Lösungsmittels und einer nicht oder nur schwach nucleophilen Base mit einem aromatischen Alkohol oder Thioalkohol der allgemeinen Formel IV
H-Ar IV
umsetzt.

6. Verfahren zur Herstellung von Perylen-3,4:9,10-tetracarbonsäurediimiden der allgemeinen Formel Ib 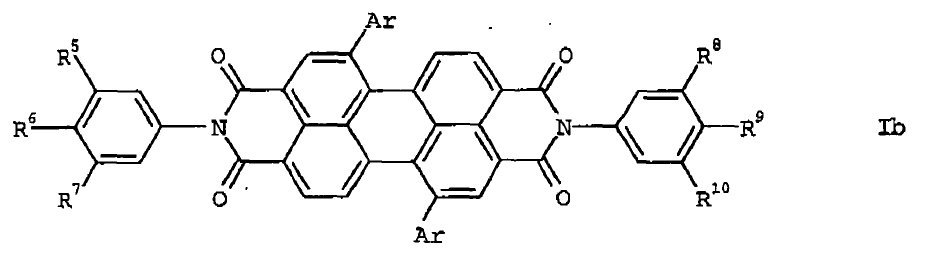 in der Ar gewünschtenfalls substituiertes Aryloxy, Arylthio, Hetaryloxy oder Heterarylthio bedeutet, **dadurch gekennzeichnet; daß** man 1,7-Dibromperylen-3,4:9,10-tetrecarbonsäuredianhydrid (IIb) in Gegenwart eines polaren aprotischen Lösungsmittels und eines Imidierungskatalysators mit gleichen oder unterschiedlichen primären Aminen der allgemeinen Formeln III 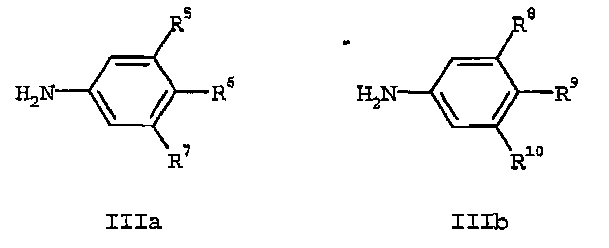 umsetzt und die dabei gebildeten 1,7-Dibromperylen-3,4:9,10-tetracarbonsäurediimide der allgemeinen Formel I" 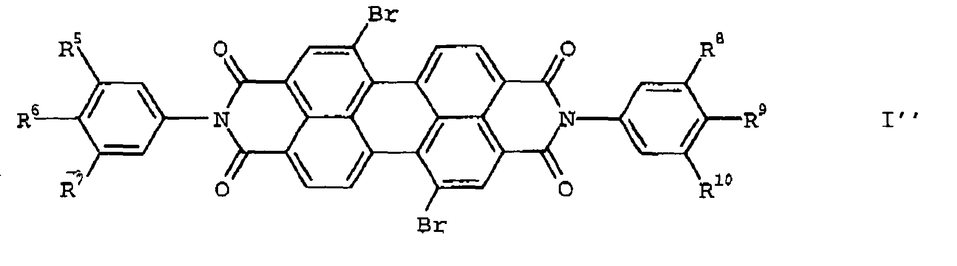 in Gegenwart eines inerten aprotischen Lösungsmittels und einer nicht oder nur schwach nucleophilen Base mit einem aromatischen Alkohol oder Thioalkohol der allgemeinen Formel IV
H-Ar IV
umsetzt.

7. Verwendung von Perylen-3,4:9,10-tetracarbonsäurediimiden der Formel I gemäß den Ansprüchen 1 bis 3 als flüssigkristalline Materialien für elektronische, optoelektronische und photonische Anwendungen.

8. Verwendung von Perylen-3,4:9,10-tetracarbonsäurediimiden der Formel I gemäß den Ansprüchen 1 bis 3 zum Einfärben von hochmolekularen organischen und von anorganischen Materialien, als Fluoreszenzfarbstoffe und als Laserfarbstoffe.

9. Verwendung von Perlrlen-3,4:9,10-tetracarbonsäurediimiden der Formel I gemäß den Ansprüchen 1 bis 3 als organische Materialien für Solarkollektoren und Elektrolumineszenzanwendungen.

## Claims

1. Perylene-3,4:9,10-tetracarboxylic diimides of the general formula I 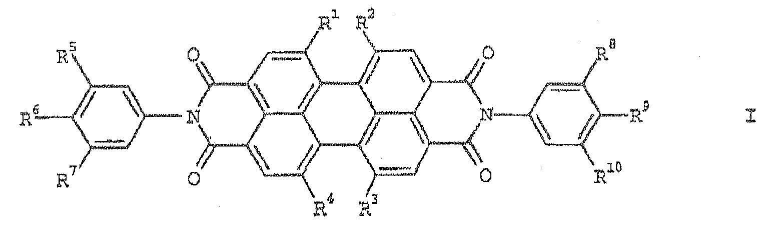 where
R¹, R², R³ and R⁴ are independently hydrogen, chlorine, bromine or substituted or unsubstituted aryloxy, arylthio, arylamino, hetaryloxy or hetarylthio;
R⁵, R⁶, R⁷, R⁸, R⁹ and R¹⁰ are independently hydrogen or C₈-C₂₀-alkyl, C₈-C₂₀-alkoxy or C₈-C₂₀-alkylthio whose carbon chain may in each case contain up to four double bonds, with the proviso that at least four of these radicals are not hydrogen.

2. Perylene-3,4:9,10-tetracarboxylic diimides according to claim 1 of the formula I where
R¹, R², R³ and R⁴ are independently hydrogen, chlorine, bromine or phenoxy which may be substituted by up to three of the following substituents: C₁-C₂₀-alkyl whose carbon chain may be interrupted by one or more moieties selected from the group consisting of -O-, -S-, -NR¹¹-, -CO- and -SO₂- and/or which may be substituted by one or more substituents selected from the group consisting of -COOR¹¹, -SO₃R¹¹, hydroxyl, cyano, C₁-C₆-alkoxy, C₅-C₈-cycloalkyl and a 5- to 7-membered heterocyclic radical which is attached by a nitrogen atom and which may contain further heteroatoms; C₁-C₆-alkoxy; cyano; hydroxyl; halogen; nitro, -COOR¹¹ or -SO₃R¹¹;
R⁵, R⁶, R⁷, R⁸, R⁹ and R¹⁰ are independently C₈-C₂₀-alkyl, C₈-C₂₀-alkoxy or C₈-C₂₀-alkylthio whose carbon chain may in each case contain up to four double bonds;
R¹¹ is hydrogen or C₁-C₆-alkyl.

3. Perylene-3,4:9,10-tetracarboxylic diimides accoring to claim 1 of the formula I where
R¹, R², R³ and R⁴ are independently hydrogen or phenoxy which may be monosubstituted by C₁-C₈-alkyl;
R⁵, R⁶, R⁷, R⁸, R⁹ and R¹⁰ are each C₁₀-C₁₄-alkyl.

4. A process for preparing perylene-3,4:9,10-tetracarboxylic diimides of the formula I as set forth in any of claims 1 to 3, which comprises reacting a perylene-3,4:9,10-tetracarboxylic dianhydride of the general formula II 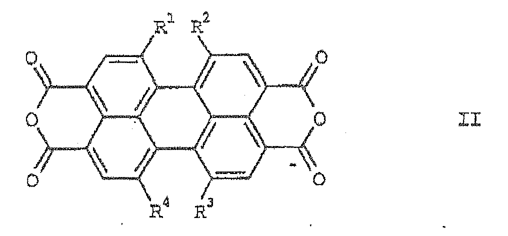 with identical or different primary amines of the general formulae III 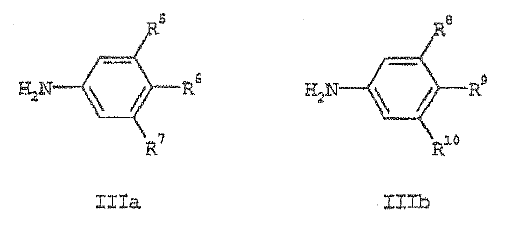 in the presence of a polar aprotic solvent and of an imidation catalyst.

5. A process for preparing perylene-3,4:9,10-tetracarboxylic diimides of the general formula Ia 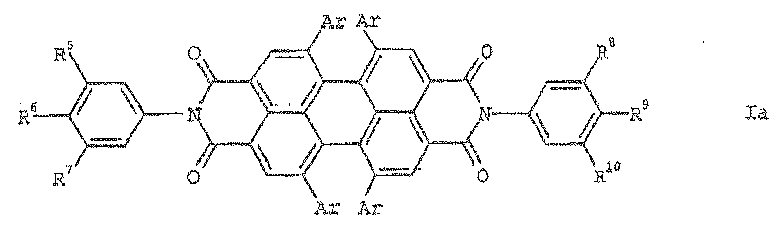 where Ar is substituted or unsubstituted aryloxy, arylthio, hetaryloxy or hetarylthio, which comprises reacting 1,6,7,12-tetrachloroperylene-3,4:9,10-tetracarboxylic dianhydride (IIa) with identical or different primary amines of the general formulae III 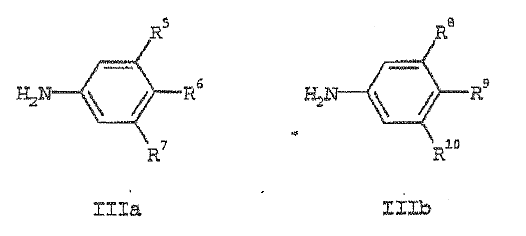 in the presence of a polar aprotic solvent and of an imidation catalyst and reacting the resultant 1,6,7,12-tetrachloroperylene-3,4:9,10-tetracarboxylic diimides of the general formula I' 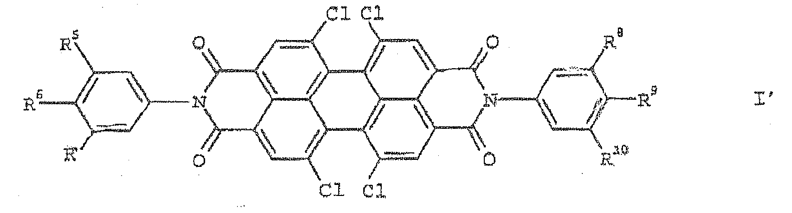 with an aromatic alcohol or thioalcohol of the general formula IV
H-Ar IV
in the presence of an inert aprotic solvent and of a non-nucleophilic or only minimally nucleophilic base.

6. A process for preparing perylene-3,4:9,10-tetracarboxylic diimides of the general formula Ib 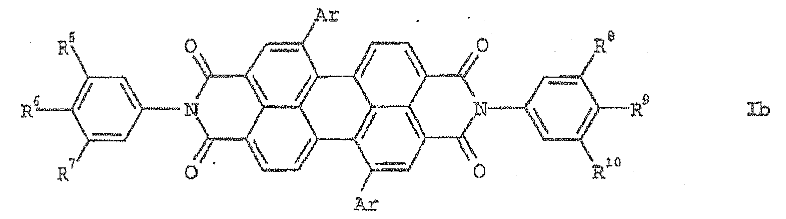 where Ar is substituted or unsubstituted aryloxy, arylthio, hetaryloxy or hetarylthio, which comprises reacting 1,7-dibromoperylene-3,4:9,10-tetracarboxylic dianhydride (IIb) with identical or different primary amines of the general formulae III 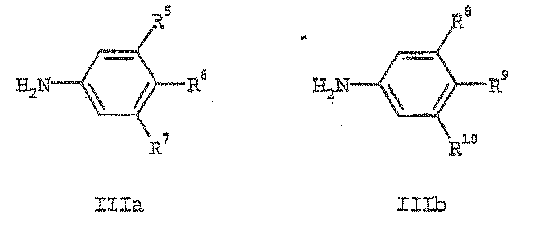 in the presence of a polar aprotic solvent and of an imidation catalyst and reacting the resultant 1,7-dibromoperylene-3,4:9,10-tetracarboxylic diimides of the general formula I'' 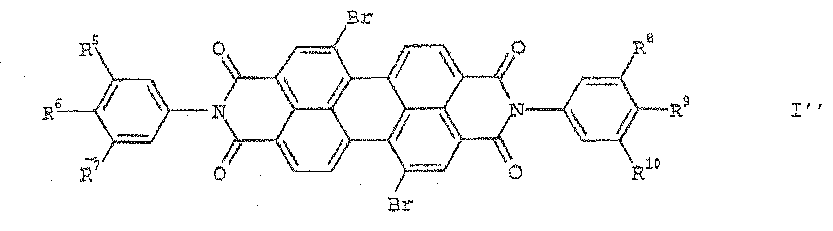 with an aromatic alcohol or thioalcohol of the general formula IV
H-Ar IV
in the presence of an inert aprotic solvent and of a non-nucleophilic or only minimally nucleophilic base.

7. The use of perylene-3,4:9,10-tetracarboxylic diimides of the formula I as set forth in any of claims 1 to 3 as liquid-crystalline materials for electronic, optoelectronic and photonic applications.

8. The use of perylene-3,4:9,10-tetracarboxylic diimides of the formula I as set forth in any of claims 1 to 3 for coloration of macromolecular organic and of inorganic materials, as fluorescent dyes and as laser dyes.

9. The use of perylene-3,4:9,10-tetracarboxylic diimides of the formula I as set forth in any of claims 1 to 3 as organic materials for solar collectors and electroluminescence applications.

## Revendications

1. Diimides d'acide pérylène-3,4:9,10-tétracarboxylique de formule générale I 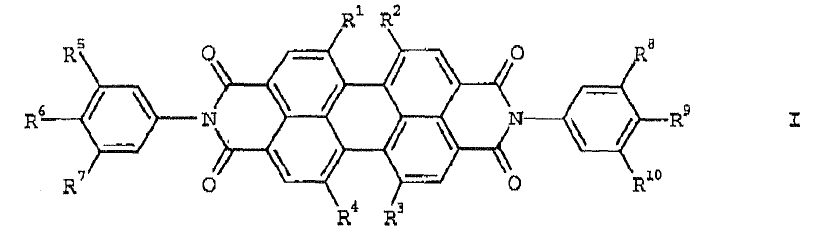 dans laquelle les variables ont la signification suivant
R¹, R², R³ et R⁴, indépendamment les uns des autres, l'hydrogène, le chlore, le brome, ou un aryloxy, un arylthio, un arylamino, un hétaryloxy ou un hétarylthio éventuellement substitué;
R⁵, R⁶, R⁷, R⁸, R⁹ et R¹⁰, indépendamment les uns des autres, l'hydrogène ou un alkyle en C₈-C₂₀, un alcoxy en C₈-C₂₀ ou un alkylthio en C₈-C₂₀ dont la chaîne de carbone peut contenir jusqu'à quatre liaisons doubles, au moins quatre de ces radicaux étant différents de l'hydrogène.

2. Diimides d'acide pérylène-3,4:9,10-tétracarboxylique de formule I selon la revendication 1, dans laquelle les variables ont la signification suivant
R¹, R², R³ et R⁴ indépendamment les uns des autres, l'hydrogène, le chlore, le brome ou un phénoxy qui peut porter jusque trois des substituants suivants : alkyle en C₁-C₂₀ dont la chaîne de carbone peut être interrompue par un ou plusieurs groupements -O-, -S-, -NR¹¹-, -CO- et/ou -SO₂- et/ou qui peut être une ou plusieurs fois substitué par un -COOR¹¹, -SO₃R¹¹, un hydroxy, un cyano, un alcoxy en C₁-C₆, un cycloalkyle en C₅-C₈ ou un radical hétérocyclique de 5 à 7 membres lié à un atome d'azote, lequel peut contenir d'autres hétéroatomes; alcoxy en C₁-C₆; un cyano; un hydroxy; un halogène; un nitro, -COOR¹¹ ou -SO₃R¹¹;
R⁵, R⁶, R⁷, R⁸, R⁹ et R¹⁰, indépendamment les uns des autres, alkyle en C₈-C₂₀, alcoxy en C₈-C₂₀ ou alkylthio en C₈-C₂₀, dont la chaîne de carbone peut contenir chaque fois jusqu'à quatre liaisons doubles;
R¹¹, l'hydrogène ou un alkyle en C₁-C_{6.}

3. Diimides d'acide pérylène-3,4:9,10-tétracarboxylique de formule I selon la revendication 1, dans laquelle les variables ont la signification suivante :
R¹, R², R³ et R⁴, indépendamment les uns des autres, l'hydrogène ou un phénoxy, qui peut être une fois substitué par un alkyle en C₁-C₈;
R⁵, R⁶, R⁷, R⁸, R⁹ et R¹⁰ un alkyle en C₁₀-C₁₄.

4. Procédé de préparation de diimides d'acide pérylène-3,4:9,10-tétracarboxylique de formule I selon les revendications 1 à 3, **caractérisé en ce qu'**on fait réagir un dianhydride d'acide pérylène-3,4:9,10-tétracarboxylique de formule générale II 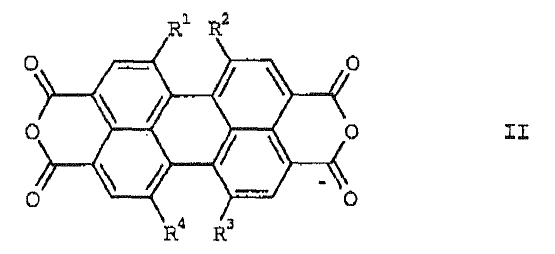 en présence d'un solvant aprotique polaire et d'un catalyseur d'imidisation avec des amines primaires identiques ou différentes de formules générales III

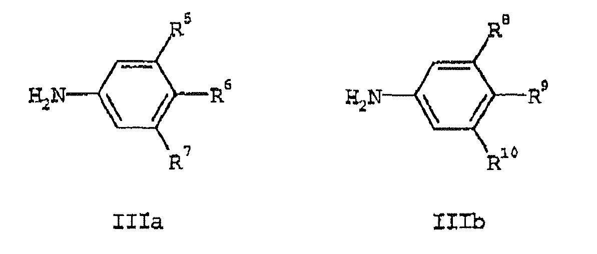

5. Procédé de préparation de diimides d'acide pérylène-3,4:9,10-tétracarboxylique de formule générale Ia 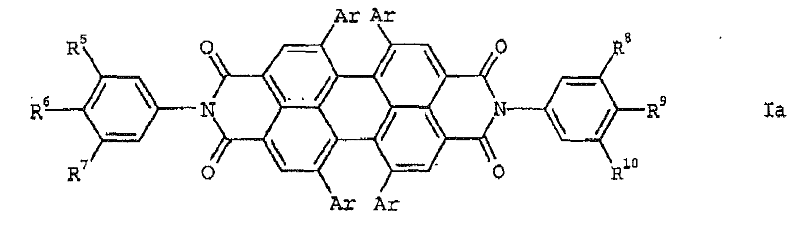 dans laquelle Ar signifie un aryloxy, un arylthio, un hétaryloxy ou un hétérarylthio éventuellement substitué, **caractérisé en ce qu'**on fait réagir un dianhydride d'acide 1,6,7,12-tétrachloropérylène-3,4:9,10-tétracarboxylique (IIa) en présence d'un solvant aprotique polaire et d'un catalyseur d'imidisation avec des amines primaires identiques ou différentes de formules générales III 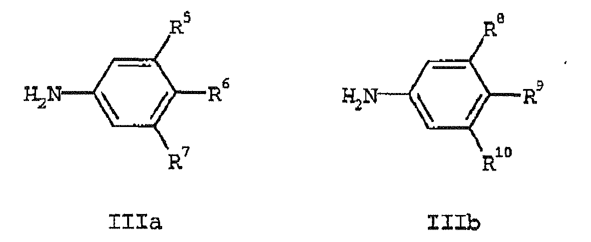 et on fait réagir les diimides d'acide 1,6,7,12-tétrachloropérylène-3,4:9,10-tétracarboxylique de formule générale I' formés à cette occasion 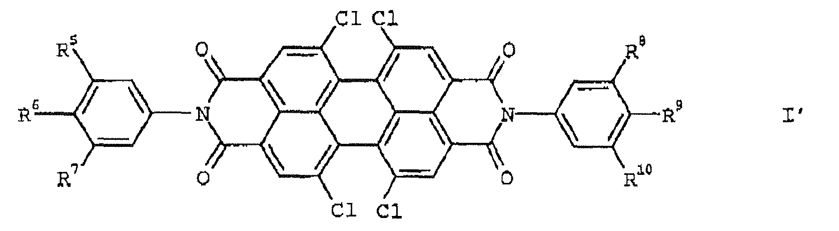 en présence d'un solvant aprotique inerte et d'une base non ou seulement peu nucléophile avec un alcool aromatique ou un thioalcool de formule générale IV
H-Ar IV

6. Procédé de préparation de diimides d'acide pérylène-3,4:9,10-tétracarboxylique de formule générale Ib 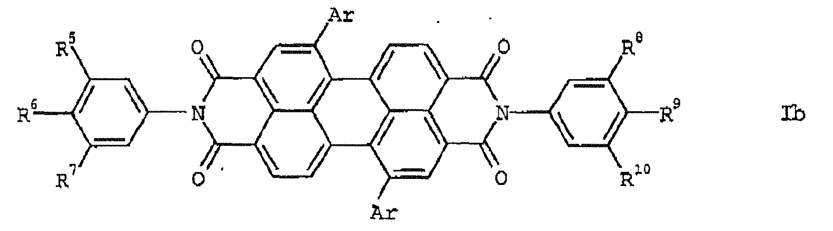 dans laquelle Ar signifie un aryloxy, un arylthio, un hétaryloxy ou un hétérarylthio éventuellement substitué, **caractérisé en ce qu'**on fait réagir un dianhydride d'acide 1,7-Dibromopérylène-3,4:9,10-tétracarboxylique (IIb) en présence d'un solvant aprotique polaire et d'un catalyseur d'imidisation avec des amines primaires identiques ou différentes de formules générales III 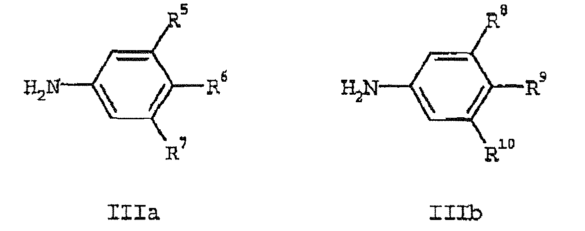 et on fait réagir les diimides d'acide 1,7-dibromopérylène-3,4:9,10-tétracarboxylique de formule générale I" formés à cette occasion 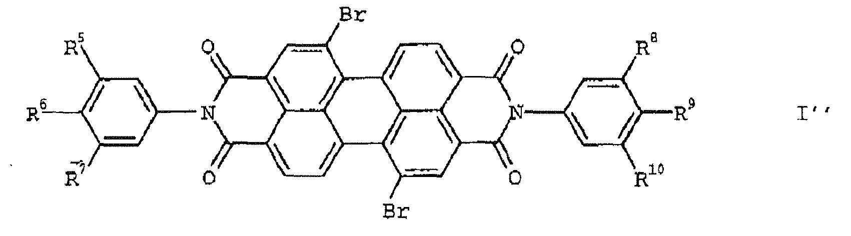 en présence d'un solvant aprotique inerte et d'une base non ou seulement peu nucléophile avec un alcool aromatique ou un thioalcool de formule générale IV
H-Ar IV

7. Utilisation de diimides d'acide pérylène-3,4:9,10-tétracarboxylique de formule I selon les revendications 1 à 3 comme matières liquides-cristallines pour des applications électroniques, opto-électroniques et photoniques.

8. Utilisation de diimides d'acide pérylène-3,4:9,10-tétracarboxylique de formule I selon les revendications 1 à 3 pour la coloration de matières organiques et inorganiques à poids moléculaire élevé, comme colorants de fluorescence et comme colorants laser.

9. Utilisation de diimides d'acide pérylène-3,4:9,10-tétracarboxylique de formule I selon les revendications 1 à 3 comme matières organiques pour des collecteurs solaires et des applications d'électroluminescence.
